Europäisches Patentamt

(19) European Patent Office   (11) Publication number: **0 100 395**

Office européen des brevets   **B1**

(12)   **EUROPEAN PATENT SPECIFICATION**

(45) Date of publication of patent specification: **01.06.88**   (51) Int. Cl.⁴: **G 01 N 33/556,** G 01 N 33/68

(21) Application number: **83101963.3**

(22) Date of filing: **28.02.83**

(54) **Reagent for determination of human urine kallikrein.**

(30) Priority: **02.08.82 JP 135001/82**

(43) Date of publication of application:
**15.02.84 Bulletin 84/07**

(45) Publication of the grant of the patent:
**01.06.88 Bulletin 88/22**

(84) Designated Contracting States:
**BE DE FR GB LU NL SE**

(56) References cited:
**DE-A-2 310 964**
**US-A-4 107 287**
**US-A-4 320 111**

**JOURNAL OF IMMUNOLOGY, vol. 126, no. 6th
June 1981, Baltimore, Maryland, USA; N.B.
OZA et al. "Antibody that recognizes total
human urinary kallikrein", pages 2361-2364**

(73) Proprietor: **THE GREEN CROSS CORPORATION
15-1, Imabashi-1-chome Higashi-ku Osaka-shi
Osaka 541 (JP)**

(72) Inventor: **Moriya, Hiroshi
14, Nishiochiai-2-chome Shinjuku-ku
Tokyo (JP)**
Inventor: **Sako, Eiji
12-9, Nagaremachi-4-chome
Hirano-ku Osaka (JP)**
Inventor: **Funakoshi, Satoshi
16-5, Aoyama-1-chome
Katano-shi (JP)**
Inventor: **Tajima, Masakazu
14-4, Terahata-1-chome
Kawanishi-shi (JP)**
Inventor: **Morichi, Shigehiro
3-18-108, Tsurukabuto-4-chome
Naka-ku Kobe (JP)**

(74) Representative: **Vossius & Partner
Siebertstrasse 4 P.O. Box 86 07 67
D-8000 München 86 (DE)**

## 0 100 395

**Description**

This invention relates to a reagent for determination of human urine kallikrein which utilizes the reversed passive hemagglutination reaction and enables a rapid and accurate quantitative determination of urine kallikrein.

Kallikrein is an enzyme which acts upon kininogen in plasma and liberates kinin which is a physiologically active peptide.

The biological action of kallikrein are developed through the liberated kinin. These actions include vasodilation, blood pressure lowering, smooth muscle contraction, enhancement of capillary vessel permeability, catecholamine secretion raising, and prostaglandin biosynthesis acceleration.

It is known that kallikrein is excreted not only in human urine but in the urine of many animals. The kallikrein in urine is produced by biosynthesis in the kidney, and participates particularly in the control of renal blood flow and blood pressure.

Thus, kallikrein plays an important role in maintaining the constancy of a living body, showing a broad range of physiological activities. Accordingly, the decrease of biosynthesis or secretion of kallikrein causes serious disease. It is known, for instance, that the amount of kallikrein excreted in the urine of a patient suffering from essential or nephrogenous hypertension is distinctly low compared with that of a normal person, and that the amount of kallikrein excreted in urine is large in cases of primary aldosteronism or Bartter's syndrome and is small in case of renal insufficiency.

Thus, in treating various diseases including hypertension, it is highly useful for the diagnosis of the disease to quantitatively determine the kallikrein excreted in urine.

For quantitative determination of kallikrein there has been used for a long time the method of Frey [Arch. klin. Chir., *142*, 663 (1926)], in which the degree of lowering of the whole blood pressure of an anesthetized dog observed when the dog is administered intravenously with a specimen is used as an index of the amount of kallikrein in the specimen. However, some problems arise with this method, such as complicated operations and difficulty in eliminating the fluctuation due to the individual differences among dogs.

In another method of determination, measurement is made for the hydrolytic activity of kallikrein on various synthetic substrates such as methyl ester of tosylarginine, ethyl ester of benzoylarginine, prolylphenylalanyl-arginine methylcoumarinamide, and D-valyl-leucyl-arginine para-nitroanilide. This method is simple in operation and high in reproducibility compared with the method of Frey. In the determination of kallikrein in urine, however, it is not possible to determine kallikrein selectively and accurately by this method because these synthetic substrates are decomposed also with proteolytic enzymes other than kallikrein present in urine.

In contrast to these methods, by application of an immunoassay using antigen-antibody reaction wherein kallikrein labelled with a radioactive substance and urine specimen act as antigens and anti-kallikrein antibody acts as antibody, it is possible to determine kallikrein selectively even when urine is the specimen to be examined. Such immunological methods of determination have been disclosed in the following reports:

J. Clin. Endocrinol, Metab., *51*, 840 (1980);

Agents and actions, *10*, 329 (1980); and

J. Immunology, *126*, 2361 (1981).

As is apparent from these reports, by the application of immunoassay, an accurate determination of trace amounts of kallikrein excreted in urine has become possible, and the correlation between various diseases and the amounts of kallikrein excreted in urine has come to be investigated in detail.

However, in the methods disclosed in above references regarding immunoassay of killikrein, the following problems are pointed out. (1) Since these methods utilize a method of labelling human urine kallikrein which is an antigen with a radioisotope in order to trace the antigen-antibody reaction quantitatively, they have their own defects originating from the radioisotope. For example, radioisotopes are generally unstable and hence a labelled compound cannot be stored for a long period of time. Further, radioisotopes are harmful to the human body, and facilities authorized under established criteria are necessary for their treatment. Moreover, the radioisotopes are expensive, and also the prices of measuring instruments are high. (2) The antigen-antibody reaction is effected in a liquid phase, and for the purpose of separating antigen (B-body), which has formed a complex with antibody, from free antigen (F-body) (so called B/F separation), the double antibody method or polyethylene glycol precipitation method is used. For this reason, incubation or centrifugation is necessary, and hence the time required for measurement is lengthened and the operations are complicated.

The objects of this invention are to solve these problems accompanying the prior methods and to provide a reagent for rapid and accurate quantitative determination of kallikrein in a large number of urine specimens.

This invention provides a reagent for determination of human urine kallikrein for reversed passive hemagglutination reaction which contains anti-human urine kallikrein-sensitized erythrocytes obtainable by sensitizing animal erythrocytes with a specific anti-human urine kallikrein antibody.

This invention provides further a kit for determination of human urine kallikrein for reversed passive hemagglutination characterized by a combination of (a) anti-human urine kallikrein sensitized erythrocytes

2

0 100 395

obtainable by sensitizing animal erythrocytes with a specific anti-human urine kallikrein antibody (hereinafter referred merely to as sensitized erythrocytes), (b) buffer solution (c) standard human urine positive control (hereinafter referred to as positive control), and (d) standard human urine negative control (hereinafter referred to as negative control) and also a method for quantitative determination of human kallikrein employing the said kit.

The essential parts of sensitized erythrocytes are sensitized erythrocytes obtainable by sensitizing animal erythrocytes with a specific anti-human urine kallikrein antibody. The specific anti-human urine kallikrein may be prepared by purifying the antiserum obtained from an animal immunized with highly purified human urine kallikrein usable for immunization. The preparation of said antiserum may be performed in a known manner. For example, a mixed emulsion prepared from highly purified human urine kallikrein and Freund's complete adjuvant is injected subcutaneously 2 to 3 times to an animal. After several days, blood is collected and is coagulated at room temperature, and after standing overnight at 4°C, is centrifuged at 3000 rpm for 20 minutes to give said antiserum as supernatant. Since the capacity of producing antibody against human urine kallikrein varies depending on animals used for immunization, it is advantageous economically to select a group of animals having high sensitivity, such as guinea pig, rabbit, sheep and domestic fowl. The preparation of specific anti-human urine kallikrein by purification of said antiserum is conducted by a method disclosed, for example, in J. Am. Chem. Soc. *62*, 3386 (1940) or Fed. Proc. *13*, 1116 (1958) (See Example 1).

The selection of the animal whose erythrocytes are to be immunized with anti-human urine kallikrein antibody is not critical, but for preparation of a stable and highly sensitive reagent, erythrocytes of sheep, domestic fowl or group O human erythrocytes are preferred. The erythrocytes are washed thoroughly with physiological saline, and then stabilized by treating with glutaraldehyde, formaline and the like. These erythrocytes have preferably a size of about 5—15 μm.

The sensitization of these erythrocytes with a purified antibody can be performed in a known method [see Igaku-no-ayumi (Progress in Medicine), *78*, 759 (1970)]. Thus, the sensitization of erythrocytes with antibody is preferably conducted in a buffer solution, and is usually effected by mixing a suspension of erythrocytes with a fluid containing antibody. This operation is preferably carried out at a pH of 6.8 to 8.5 and a temperature of 20° to 60°C. Preferably, the sensitized erythrocytes are lyophilized, loaded in a vessel such as a vial and sealed preferably with an added preservative such as sodium azide.

The positive control included in this invention is used for the preparation of standard calibration line, and usually comprises a lyophilized product containing human urine kallikrein. Examples of such human urine kallikrein to be used include a lyophilized human urine kallikrein obtained, for instance, by a method in which fresh human urine is contacted with colloidal hydrated aluminium silicate and then subjected to affinity chromatography with a insoluble trypsin inhibitor. The human urine kallikrein is dissolved in a buffer solution in 4 to 6 steps of concentrations ranging, for example, from 5 to 50 ng/ml, and is used for preparation of the standard calibration line.

The positive control is loaded in a vessel such as a vial and preferably contains a preservative such as sodium azide.

The negative control comprises a liquid (such as human serum or physiological saline) to which both human urine kallikrein antibody and human urine kallikrein antigen are negative. It is preferably loaded in a vessel such as a vial and sealed after adding some preservative, such as sodium azide.

The buffer solution is used for dilution of sensitized erythrocytes, positive control and negative control, and comprises, for example, a physiologically isotonic phosphate buffer solution having a pH of 6 to 8. In order to prevent a nonspecific reaction from occurring, there may be added, for example, stroma or animal serum. Also, some preservatives such as sodium azide are preferably added.

In the kit of this invention, there may be also added to the combination a confirmation reagent for judging the specificity of hemagglutination reaction (hereinafter referred to as confirmation reagent). The confirmation reagent comprises anti-human urine kallikrein.

Example 1

A rabbit was immunized with purified human urine kallikrein having a specific activity of 6.6 kallikrein unit/mg protein to give anti-human urine kallikrein serum. This antiserum was purified by ammonium sulfate fractionation, anion exchange resin, gel filtration or other suitable means to improve the purity of the purified human urine kallikrein antibody. Thus, this antiserum was diluted two-fold with physiological saline, mixed with an equivalent volume of saturated ammonium sulfate solution, stirred for 30 minutes, let stand for 30 minutes and then centrifuged to recover the precipitate. The precipitate was dissolved in 0.0175 M phosphate buffer solution (pH 6.3), passed through DEAE-cellulose equilibrated with said buffer solution, and purified by exhaustively eluting the residual liquid in the cellulose with said buffer solution. The eluate was dialyzed and concentrated by dialysis under reduced pressure, and then, using said buffer solution to which an amount of sodium chloride had been added to give a sodium chloride concentration equal to that of physiological saline, was subjected to gel filtration by use of a gel prepared by swelling G-200 Sephadex®. The first peak portion fractionally obtained was used for sensitization as purified human urine kallikrein antibody.

The animal erythrocytes to be sensitized were obtained by washing sheep erythrocytes thoroughly with a phosphate buffer solution (pH 6—8), adding glutaraldehyde to a final concentration of 0.5% (W/V),

# 0 100 395

treating the mixture at room temperature for about one hour, and then removing glutaraldehyde with the above buffer solution to give stabilized erythrocytes.

A suspension containing 5% (W/V) of stabilized erythrocytes was mixed with an equal amount of the above mentioned purified human urine kallikrein antibody (about 0.01 as $E_{280\ nm}$), 5—20 mg/ml of tannic acid was added thereto at pH 7.2, and after stirring, the mixture was allowed to stand overnight at 4°C. Thus, sensitization was effected to give anti-human urine kallikrein-sensitized erythrocytes. These were washed thoroughly with the above buffer solution. These sensitized erythrocytes were subdivided and put into vessels and then lyophilized to give the reagent.

The lyophilized anti-human urine kallikrein-sensitized erythrocytes were suspended in an isotonificated phosphate buffer solution (pH 7.2) containing animal serum and sodium chloride, were regulated to a concentration of 0.5% with a phosphate buffer solution containing 2% or more of sheep erythrocyte stroma, and were confirmed for their agglutination reaction against human urine kallikrein by the microplate method. The quantity of human urine kallikrein contained in a specimen could be determined at a concentration down to 2 ng/ml.

Example 2 Constitution of a kit

The present kit for determination of human urine kallikrein contains the following five kinds of reagents.

(1) Anti-human urine kallikrein sensitized sheep erythrocytes:

The content of one vial is formed of dried sensitized sheep erythrocytes and preservatives. A 0.5% (W/V) erythrocytes suspension is obtained by adding thereto 5 ml of the phosphate buffer solution (PBS) described later in (4) and suspending the content. One mg of sodium azide has been added as a preservative.

(2) Human urine kallikrein positive control:

One vial contains 1 ml of a sterilely filtered solution of human urine kallikrein. The quantity of human urine kallikrein in this solution is 1:64 or more by RPHA method. One mg (0.1%) of sodium azide has been added as preservative.

(3) Negative control:

One ml contains 1 ml of human serum or physiological saline to which sterilely filtered human urine kallikrein antibody and human urine kallikrein antigen are both negative. One mg (0.1%) of sodium azide has been added as preservative.

(4) Phosphate buffer solution

One vial contains 50 ml of a sterilely filtered phosphate buffer solution (PBS) (pH 7.2) made isotonic by addition of sodium chloride. Soluble stroma and animal serum have been incorporated into PBS to prevent a nonspecific reaction from occuring on conducting a test.

| Composition (in 50 ml) | |
|---|---|
| Disodium hydrogen phosphate (anhydrous) | 395 mg |
| Potassium dihydrogen phosphate | 155 mg |
| Sodium chloride | 225 mg |
| Stroma | 3% |
| Animal serum | 1% |
| Sodium azide | 50 mg |
| pH | 7.2 |

Ten ml of the phosphate buffer solution is used for suspending anti-human urine kallikrein sensitized-sheep erythrocytes and the remainder is used for diluting the specimen.

(5) Confirmation reagent for judging specificity

This is a lyophilized product containing sterilely filtered purified human urine kallikrein antibody (1:512 or more as PHA value) in one vial. It is dissolved in 5 ml of physiological saline.

Example 3

A quantitative test was carried out as follows by using the reagent described in Example 2.

(1) Anti-human urine kallikrein sensitized-sheep erythrocytes ① were suspended in 5 ml of the phosphate buffer solution ④ per one vial.

(2) 25 µl of the phosphate buffer solution was put in each hole of a U-plate by use of a dropper.

(3) 25 µl of sample liquid was put in each hole of the A-row by use of a diluter. The mixed liquid of the A-row was transferred to B-row by use of the diluter employed above, and then transferred from the B-row to the C-row. This procedure was repeated successively until the J-row was reached. In this way, the sample liquid was diluted in doubling dilutions, and the dilution of the liquid in the J-row was 1:1024.

4

(4) Separately, similar dilution series were prepared in the same manner from the positive control ② and the negative control ③ in place of the sample liquid.

(5) Each 25 µl of the suspension of anti-human urine kallikrein sensitized-sheep erythrocytes in phosphate buffer solution was put into every hole by use of a dropper.

(6) The plate was shaken with a micromixer for about 10 seconds, and then incubated at room temperature for 2 hours.

(7) Observation of the control

With regard to the positive control, agglutination was usually observed at 64-fold or more dilutions. In the negative control, precipitated erythrocytes were observed at the bottom of every hole of A to J row (when the measurement proceeded favorably, the negative image was small and clear).

(8) Test results

The value of the maximum dilution at which the agglutination image was observed with the test sample in comparison with the negative control was used as the human urine kallikrein value of the sample. For precise determination of kallikrein content, a standard calibration line was prepared by using solutions of human kallikrein of known concentrations and plotting on a logarithmic paper the value of dilution of said solution and the diameter (mm) of corresponding agglutination image. The content of kallikrein in an unknown sample liquid was determined from the value of dilution and the diameter of agglutination image of the sample by using the calibration line.

Example 4
Comparison on sensitivity and specificity

Comparative tests on quantitative determination of kallikrein were conducted with human urine specimen.

The results of tests conducted by the method of reversed passive hemagglutination (RPHA method) of this invention, crossed electrophoresis (CEP method) and solid phase radioimmunoassay (RIA method: a test product of Green Cross Co.) were shown in Table 1.

TABLE 1
Quantitative determination test of kallikrein in human urine specimens

| Specimen | RPHA value | RIA value | CEP value |
|----------|-----------|-----------|-----------|
| 1 | 1:4096 | 1:4096 | 1:2 |
| 2 | 1:2048 | 1:2048 | 1:1 |
| 3 | 1:1024 | 1:1024 | <1:1 |
| 4 | 1:512 | 1:512 | Negative |
| 5 | 1:16 | 1:16 | Negative |
| 6 | 1:2 | 1:2 | Negative |
| 7 | Negative | Negative | Negative |

In the specimen No. 2, for example, whilst the CEP value was 1:1, the RIA value was 1:2048, and the RPHA value of this invention showed the same sensitivity of this method as that of RIA method. A satisfactory correlation was observed between the results obtained by the RPHA method of this invention and those by the RIA method.

A separate test was conducted to examine the specificity of the present reagent with human urine specimen. When human urine specimens were reexamined after neutralized beforehand with an antigen, less than 3% thereof were judged as false positive, which showed the very high specificity of the present reagent for determination.

**Claims**

1. A reagent for determination of human urine kallikrein for reversed passive hemagglutination reaction which comprises anti-human urine kallikrein sensitized-erythrocytes obtainable by sensitizing animal erythrocytes with a specific anti-human urine kallikrein antibody.

2. A kit for determination of human urine kallikrein which comprises a combination of (a) anti-human urine kallikrein sensitized-erythrocytes obtainable by sensitizing animal erythrocytes with a specific anti-human urine kallikrein antibody, (b) buffer solution, (c) standard human urine positive control and (d) negative control.

3. The kit for determination of human urine kallikrein of Claim 2, which comprises combining further to said combination a confirmation reagent for judging the specificity of hemagglutination reaction which reagent comprises anti-human urine kallikrein antibody.

4. The kit for determination of human urine kallikrein of Claim 2 in which each of the components (a), (b), (c) and (d) contains sodium azide in an amount effective for preservation.

5. The reagent of Claim 1 in which the animal erythrocytes are selected from sheep or domestic fowl erythrocytes or group O human erythrocytes.

6. A method for determination of human urine kallikrein by reversed passive hemagglutination characterized in that anti-human urine kallikrein sensitized erythrocytes obtainable by sensitizing animal erythrocytes with specific anti-human urine kallikrein antibody are reacted with urine specimen kallikrein.

**Patentansprüche**

1. Reagenz für die Bestimmung von menschlichem Urin-Kallikrein zur umgekehrten passiven Hämagglutinations-Reaktion, das gegen menschliches Urin-Kallikrein sensitivierte Erythrozyten enthält, die durch Sensitivierung tierischer Erythrozyten mit einem spezifischen Antikörper gegen menschliches Urin-Kallikrein erhältlich sind.

2. Besteck für die Bestimmung von menschlichem Urin-Kallikrein, das eine Kombination enthält aus

a) gegen menschliches Urin-Kallikrein gerichtet sensitivierte Erythrozyten, die durch Sensitivierung tierischer Erythrozyten mit einem spezifischen Antikörper gegen menschliches Urin-Kallikrein erhältlich sind,

b) Pufferlösung,

c) standardisierter menschlicher Urin als positive Kontrolle, und

d) negative Kontrolle.

3. Besteck für die Bestimmung von menschlichem Urin-Kallikrein nach Anspruch 2, das zusätzlich zu der Kombination noch ein Bestätigungs-Reagenz zur Beurteilung der Spezifität der Hämaglutinations-Reaktion enthält, welches einen gegen menschliches Urin-Kallikrein gerichteten Antikörper enthält.

4. Besteck für die Bestimmung von menschlichem Urin-Kallikrein nach Anspruch 2, in dem jeder der Bestandteile (a), (b), (c) und (d) Natriumazid in einer zur Konservierung wirksamen Menge enthält.

5. Reagenz nach Anspruch 1, in dem die tierischen Erythrozyten Schaf- oder Hausgeflügel-Erythrozyten oder menschliche Erythrozyten der Gruppe 0 sind.

6. Verfahren zur Bestimmung von menschlichem Urin-Kallikrein durch umgekehrte passive Hämagglutination, dadurch gekennzeichnet, daß man gegen menschliches Urin-Kallikrein sensitivierte Erythrozyten, die erhältlich sind durch Sensitivierung tierischer Erythrozyten mit einem spezifischen Antikörper gegen menschliches Urin-Kallikrein, mit Kallikrein einer Urin-Probe umsetzt.

**Revendications**

1. Réactif pour la détermination de la kallikréine d'urine humaine pour la réaction d'hémagglutination passive inversée, qui comprend des érythrocytes sensibilisés à l'anti-kallikréine d'urine humaine, pouvant être obtenus en sensibilisant des érythrocytes d'animaux avec un anti-corps anti-kallikréine d'urine humaine particulier.

2. Trousse pour la détermination de la kallikréine d'urine humaine, qui comprend une combinaison (a) d'érythrocytes sensibilisés à l'anti-kallikréine d'urine humaine pouvant être obtenus en sensibilisant des érythrocytes d'animaux avec un anti-corps anti-kallikréine d'urine humaine particulier, (b) d'une solution tampon, (c) d'un témoin possitif d'urine humaine étalon et (d) d'un témoin négatif.

3. Trousse pour la détermination de la kallikréine d'urine humaine de la revendication 2, qui comprend en outre l'association à cette combinaison d'un réactif de confirmation pour juger de la spécificité de la réaction d'hémagglutination, lequel réactif comprend un anti-corps anti-kallikréine d'urine humaine.

4. Trousse pour la détermination de la kallikréine d'urine humaine de la revendication 2, dans laquelle chacun des constituants (a), (b), (c) et (d) contient de l'azide de sodium dans une quantité efficace pour la conservation.

5. Réactif de la revendication 1, dans lequel les érythrocytes d'animaux sont choisis parmi les érythrocytes de moutons ou de poules domestiques ou les érythrocytes humains du groupe 0.

6. Procédé de détermination de la kallikréine d'urine humaine par hémagglutination passive inversée, caractérisé en ce que des érythrocytes sensibilisés à l'anti-kallikréine d'urine humaine pouvant être obtenus en sensibilisant des érythrocytes d'animaux avec un anti-corps anti-kallikréine d'urine humaine sont mis à réagir avec une kallikréine d'échantillon d'urine.